(19) Europäisches Patentamt

European Patent Office

Office européen des brevets

(11) Veröffentlichungsnummer: **0 317 705 B1**

---

(12) **EUROPÄISCHE PATENTSCHRIFT**

---

(45) Veröffentlichungstag der Patentschrift: **30.09.92**

(21) Anmeldenummer: **88111725.3**

(22) Anmeldetag: **20.07.88**

(51) Int. Cl.5: **A61M 5/14**

---

(54) **Dosiergerät zum gesteuerten Injizieren von Flüssigkeiten aus einem Vorratsbehälter in einen Organismus.**

---

(30) Priorität: **25.11.87 DE 3739972**

(43) Veröffentlichungstag der Anmeldung:
**31.05.89 Patentblatt  89/22**

(45) Bekanntmachung des Hinweises auf die
Patenterteilung:
**30.09.92 Patentblatt  92/40**

(84) Benannte Vertragsstaaten:
**DE FR GB IT SE**

(56) Entgegenhaltungen:
**EP-A- 0 183 351**

(73) Patentinhaber: **SIEMENS AKTIENGESELL-
SCHAFT
Wittelsbacherplatz 2
W-8000 München 2(DE)**

(72) Erfinder: **Obermann, Peter, Dipl.-Ing.
Am Färberhof 12
W-8520 Erlangen(DE)**
Erfinder: **Franetzki, Manfred, Dr.
Heckenweg 6
W-8525 Uttenreuth(DE)**

---

Rank Xerox (UK) Business Services

**Beschreibung**

Die Erfindung betrifft ein Dosiergerät zum gesteuerten Injizieren von Flüssigkeiten aus einem Vorratsbehälter in einen Organismus nach dem Oberbegriff des Patentanspruchs 1.

Solche Dosiergeräte werden vor allem in der Diabetes-Therapie zum Injizieren von Insulin aus einem Vorratsbehälter in den Körper nach einem bestimmten Dosierungsprogramm eingesetzt. Dabei ist vorzugsweise der Pumpenteil implantierbar und der Bedienteil extrakorporal angeordnet. Die Kommunikation zwischen beiden Teilen erfolgt über eine telemetrische Fernsteuerung. Da sich die Pumpe des Dosiergerätes im Implantat befindet, muß auch die Energiequelle in Form einer Batterie implantiert werden. Zur Erreichung einer möglichst langen Lebensdauer der Batterie ist eine effiziente Energieausnutzung für den Pumpenantrieb von besonderer Bedeutung. Außerdem ist wichtig, eine Kontrolle über die von der Pumpe geförderte und injizierte Flüssigkeitsmenge zu haben, weil sonst lebensbedrohliche Zustände eintreten können.

Durch die US-PS 4 486 190 ist ein Dosiergerät der oben beschriebenen Art bekannt, dessen Funktion mit Hilfe eines Strömungswiderstandes am Pumpenausgang und eines Drucksensors in der Pumpenkammer überwacht wird. Diese für Membranpumpen brauchbare Ausführungsform ist technisch recht aufwendig und insbesondere für Kolbenpumpen, die aufgrund eines optimierten Hubraum-/Totraumverhältnisses in der Lage sind, auch Gase bei Differenzdrücken mit relativ hoher Förderrate zu pumpen, nicht brauchbar.

Solche Pumpen transportieren auch dann die zu injizierende Flüssigkeit, wenn sich in diesen Gasblasen befinden. Ein solcher Blaseneinschluß ist in der Praxis kaum vermeidbar. Pumpen dieser Art sind in Gestalt von Kolbenpumpen in der EP-A-0259668 und in der DE-A-37 13 498.1 beschrieben.

Eine weitere Überwachungsanordnung für eine implantierte Kolbenpumpe eines Medikamenten-Dosiergerätes ist aus der AT-B-378 123 bekannt. Dabei wird das in den Antriebsspulen des Kolbens der Kolbenpumpe bei dessen Bewegung entstehende Mikrophoniegeräusch registriert und bei dessen Fehlen ein Alarmsignal ausgelöst. Eine Überwachung der Pumpe auf Funktionsabweichungen von Sollwerten mit Auslösung von Alarmsignalen oder Steuerbefehlen im Falle von Abweichungen ist dabei jedoch nicht vorgesehen.

Eine weitere Überwachungsanordnung für eine implantierte Infusionspumpe eines Medikamenten-Dosiergerätes ist aus der EP-A-0 048 423 bekannt. Dabei geschieht die Überwachung in der Weise, daß die in den Pumpenantrieb eingegebenen elektrischen Betriebsimpulse auf direkte oder indirekte Weise drahtlos an das extern angeordnete Steuer- bzw. Programmiergerät zurückgemeldet und damit der Empfang dieser Impulse bestätigt wird. Ob diese Impulse tatsächlich zu einer Pumpbewegung geführt haben und ob diese Pumpbewegung die gewünschte Medikamentenmenge gefördert hat, ist mit dieser Anordnung jedoch nicht feststellbar.

Schließlich ist es aus der DE-A-2 916 490 bekannt, bei einem Kolbenkompressor für verfahrenstechnische Anlagen, z.B. zum Verdichten von Äthylengas, die Funktion von dessen Ventilen durch Abnahme der auf den Zylinderkopf übertragenen Schallwellen mit Hilfe von Schwingungsaufnehmern zu überwachen, mit Sollwerten zu vergleichen und bei Fehlfunktionen ein Alarm- oder Ausschaltsignal auszulösen. Mittel für die Überwachung und Analyse des zeitabhängigen Bewegungsverlaufes eines Kolbens sind auch dieser Druckschrift nicht zu entnehmen.

Der Erfindung liegt die Aufgabe zugrunde, ein Dosiergerät der eingangs beschriebenen Art so zu gestalten, daß die Pumpfunktion auf indirekte Weise mit einfachen elektrischen oder mechanischen Mitteln erfaßt werden kann und die dabei gewonnenen zeitabhängigen Verlaufswerte durch eine Analyse des Kurvenverlaufs kontrolliert und daraus Steuerungs- bzw. Anzeigewerte gewonnen werden können.

Diese Aufgabe wird durch die im Patentanspruch 1 angegebene Erfindung gelöst. Dadurch ist insbesondere erreicht, daß Anfang und Ende des Kolbenhubs der Pumpe eindeutig ermittelt und damit die Energiezufuhr zur Statorwicklung optimal gesteuert werden kann. Darüber hinaus können aus der Analyse des Bewegungsverlaufs des Kolbens Informationen abgeleitet werden, die nach außen geführt und als Funktionskontrolle mit Alarmauslösung bei kritischen Zuständen eingesetzt werden können.

Weitere Einzelheiten der Erfindung sind in den Unteransprüchen gekennzeichnet. Dabei zeichnet sich die Lösung nach Patentanspruch 4 dadurch aus, daß der Bewegungsverlauf des Kolbens mit rein elektrischen Mitteln und daher besonders raumsparend erfaßt werden kann.

Die Ausführungsform nach Patentanspruch 7 hat den Vorteil, daß auch Funktionsstörungen im mechanischen Teil der Pumpe, wie etwa unzureichender Medikamententransport, erfaßt werden können.

Anhand von Ausführungsbeispielen wird die Erfindung im folgenden näher erläutert. Es zeigen:

FIG 1    einen Querschnitt durch den mechanischen Teil der Pumpe;

FIG 2    den elektronischen Teil mit Steuer- und Überwachungsanordnung sowie dem über eine Telemetriestrecke damit in Fernwirkverbindung stehenden Programmier-, Anzeige- und Überwachungsgerät;

FIG 3 eine andere Ausführungsform der Pumpe gemäß FIG 1;

FIG 4 eine der Pumpe nach FIG 3 zugeordnete Steuer- und Überwachungsanordnung mit dazu in Telemetrieverbindung stehendem Programmier-, Anzeige- und Überwachungsgerät;

FIG 5 einen typischen Kurvenverlauf des durch die Statorwicklung fließenden Aktionsstromes für die Kolbenbewegung der Pumpe und

FIG 6 eine andere Ausführungsform der Steuer- und Überwachungsanordnung nach FIG 4.

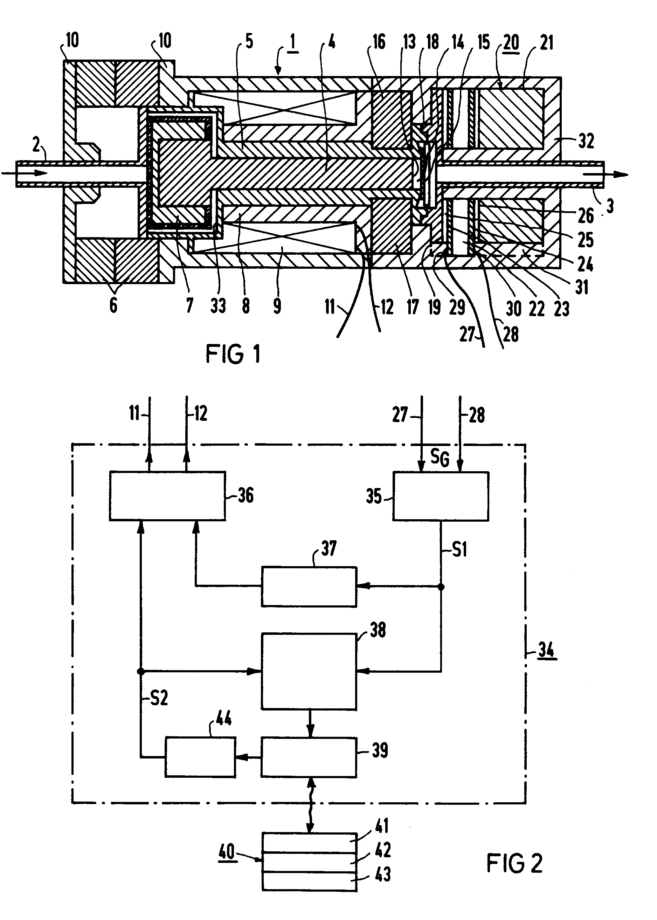

Die in FIG 1 als Querschnitt dargestellte Ausführungsform einer Kolbenpumpe 1 eines Flüssigkeits-Dosiergerätes umfaßt einen Einlaß 2 und einen Auslaß 3 für die in einen Organismus zu injizierende Flüssigkeit, beispielsweise Insulin. Sie besitzt ferner einen Kolben 4, der in einem Zylindergehäuse 5 untergebracht ist. Das Kolbenantriebssystem umfaßt Permanentmagneten 6, einen gekapselten Anker 7 und einen Stator 8 mit Statorwicklung 9. Die Polschuhe der Permanentmagnete 6 bzw. des Stators 8 sind mit 10 bezeichnet. Die Statorwicklung 9 ist über die Anschlußleitungen 11, 12 mit einer nicht dargestellten Energiequelle sowie einer noch zu beschreibenden Steuer- und Überwachungsanordnung verbunden. Die Kolbenstirnfläche 13 begrenzt den Kolbenhubraum, der gebildet ist vom umgebenden Zylindergehäuse 5 und dem Ventil 14 in Kolbenrichtung. Beim Auftreten einer Antriebskraft durch Erregung der Statorwicklung 9 bewegt sich der Kolben 4 in Richtung auf das Ventil 15 und preßt dabei mit seiner Stirnfläche 13 das im Kolbenhubraum 14 befindliche Medikament durch das Ventil 15 gegen die Rückstellkraft der Permanentmagnete 16, 17 in den Auslaßkanal 3 und von diesem über einen nicht dargestellten Katheter, an dessen Spitze eine in ein Körpergefäß eingeführte Injektionskanüle angeordnet ist, in dieses Gefäß. Nach dem Anstoßen der Kolbenstirnfläche 13 an die innere Ventilwand und dem Ende des Flüssigkeitsdrucks schließt das Ventil durch die von den Permanentmagneten 16, 17 ausgeübte Rückstellkraft. An der Auslaßseite des Flansches 19 der Kolbenpumpe 1 ist, konzentrisch zum Auslaß 3, ein Geräuschsensor 20 aufgesetzt. Dieser umfaßt eine mittels einer metallischen Koppelmasse 21 (z.B. aus Messing oder Stahl) als Bezugsmasse verbundene piezokeramische Ringscheibe 22. Diese beiden Metallschichten 23, 24 dienen als Elektroden. Auf jede dieser Metallschichten ist eine Isolierschicht 25, 26 aus Epoxydharz aufgebracht. Diese beiden Isolierschichten dienen gleichzeitig als Kleber zur Befestigung des Geräuschsensors 20 am Flansch 19 einerseits und zur Befestigung der Koppelmasse 21 an der piezokeramischen Ringscheibe 22 andererseits. Die Metallschichten 23, 24 sind an den Pumpen 29, 30 mit Ausgangsleitungen 27, 28 verbunden, die in Vertiefungen 31 des Flansches 19 und der Koppelmasse 21 nach außen geführt sind. Zur mechanischen Stabilisierung ist schließlich der Geräuschsensor 20 in einem Gehäuse 32 aus Epoxydharz-Gießmasse angeordnet, das an der Kolbenpumpe 1 fixiert ist. Der mit dem Kolben 4 fest verbundene Anker 7 ist so ausgebildet, daß dessen ausgangsseitige ringförmige Fläche an der eingangsseitigen ringförmigen Fläche 33 des Zylindergehäuses 5 anliegt. Beim Aufeinandertreffen dieser beiden Flächen wird ein Anschlaggeräusch erzeugt, das sich aus dem allgemeinen Pumpgeräusch heraushebt. Dieses Anschlaggeräusch wird über das Zylindergehäuse 5 und den Flansch 19 akustisch auf dem Geräuschsensor 20 übertragen. Dieses akustische Anschlaggeräusch wird vom Geräuschsensor 20 wahrgenommen und in ein elektrisches Ausgangssignal $S_G$ umgesetzt, das sich an den Ausgangssignalleitungen 27, 28 abnehmen läßt.

In FIG 2 ist das Prinzipschaltbild einer Steuer- und Überwachungsanordnung 34 dargestellt, welches in der Lage ist, das Ausgangssignal $S_G$ des Geräuschsensors 20 so zu verarbeiten, daß dieses Anschlaggeräusch sowohl zum Steuern wie zum Überwachen der Pumpe verwendet werden kann. Dazu sind die Ausgangsleitungen 27, 28 des Geräuschsensors 20 mit dem Eingang eines Amplitudendiskriminators 35 und die Ansteuerleitungen 11, 12 der Statorwicklung 9 der Kolbenpumpe 1 mit dem Ausgang einer Pumpensteuerelektronik 36 verbunden. Der Amplituden-Schwellenwertdiskriminator 35 ist ausgangsseitig mit den Eingängen einerseits einer Abschalteinrichtung 37 und andererseits einer Fehlererfassungseinrichtung 38 verbunden. Die Fehlererfassungseinrichtung 38 ist ausgangsseitig an eine Telekommunikationselektronik 39 angeschlossen, die ihrerseits über eine drahtlose Telekommunikationsstrecke mit einem extrakorporalen Programmiergerät 40 in Verbindung steht. Ausgangsseitig ist diese Telekommunikationselektronik 39 an den Eingang einer Pumpratensteuerung 44 angeschlossen, deren Ausgangssignal $S_2$ an einen zweiten Eingang der Fehlererfassungseinrichtung 38 sowie den Eingang der Pumpsteuerelektronik 36 gelangt. Der zweite Eingang der Pumpensteuerelektronik 36 steht mit dem Ausgang der Abschalteinrichtung 37 in Verbindung. Das extrakorporale Programmiergerät 40 besitzt seinerseits eine Telekommunikationselektronik 41, einen Bedienteil 42 und einen Anzeigeteil 43 für fehlerhafte Funktionszustände, die in Form einer Alarmanzeige akustisch, optisch und/oder sensorisch durch subkutanen Reizstrom dargestellt werden können. Die Pumpratensteuerung 44 ist vom Programmiergerät 40 mit Hilfe des Bedienteils 42 über die Telekommunikationsstrecke programmierbar.

Während eines jeden Pumpzyklus werden bestimmte elektrische Pumpgeräusche $S_G$ erzeugt, aus denen sich die Anschlaggeräusche des gekapselten Ankers 7 an der Stirnfläche 33 des Zylindergehäuses 5 besonders hervorheben. Der Amplituden-Schwellendiskriminator 35 erzeugt nun beim Auftreten eines solchen Signals ein entsprechendes Ausgangssignal $S_1$. Dieses gelangt an die Abschalteinrichtung 37, die ein entsprechendes Steuersignal erzeugt, welches an die Pumpensteuerelektronik 36 gelangt, die den Stromkreis der Statorwicklung 9 solange unterbricht, bis von der Pumpratensteuerung 44 programmgemäß ein neues Einschaltsignal erzeugt wird. Demzufolge ist die Pumpe 1 immer exakt am Ende einer Pumpaktion abgeschaltet. Eine sicherheitshalber über das Ende einer Pumpaktion hinausgehende Einschaltung wegen fehlender Rückmeldung mit entsprechend höherem Energiebedarf ist nicht mehr notwendig.

Das an die Fehlererfassungseinrichtung 38 gelangende Ausgangssignal $S_1$ des Amplituden-Schwellenwertdiskriminators 35 wird von dieser auf Abweichungen vom typischen Verlauf hinsichtlich Amplitude und Zeit untersucht. Damit kann beispielsweise festgestellt werden, ob die Pumpe evtl. blockiert ist, ob ein Blasentransport stattfindet, ob eine Nichtförderung aufgrund eines geleerten Medikamenten-Vorratsbehälters oder eine beginnende Katheterverstopfung vorliegt. Dazu wird das Ausgangssignal $S_2$ der Pumpratensteuerung 44 mit dem Ausgangssignal $S_1$ des Amplituden-Schwellenwertdiskriminators 35 in der Fehlererfassungseinrichtung 38 zeitlich korreliert. Dabei ergeben sich im Falle von Signalabweichungen durch Fehlfunktionen unterschiedliche Signalzustände. Bei einer Blockade etwa des Katheters entsteht gar kein Ausgangssignal $S_1$ innerhalb des Korrelationszeitintervalls, während sich bei Nichtförderung aufgrund eines geleerten Vorratsbehälters bzw. bei Blasentransport ein stark verfrühtes bzw. schwach verfrühtes Ausgangssignal $S_1$ einstellt. Bei einer beginnenden Katheterverstopfung erhält man durch den dabei steigenden Katheterströmungswiderstand bei niedriger Pumprate (Basalrate) noch Signale $S_1$, die beim Umschalten auf hohe Raten (Abrufraten) jedoch verschwinden. Die Art des Fehlers wird von der Fehlererfassungseinrichtung 38 über die Telekommunikationsstrecke, beispielsweise beim nächsten Programmiervorgang, an die Anzeige 43 des extrakorporalen Programmiergerätes 40 übermittelt und dort optisch, akustisch und/oder sensorisch angezeigt. Dem Träger des Dosiergerätes wird durch diese Art der Information, die evtl. mit einem Alarmsignal gekoppelt ist, ermöglicht, frühzeitig geeignete therapeutische Maßnahmen sowie auch ggf. Maßnahmen zur Fehlerbeseitigung einzuleiten.

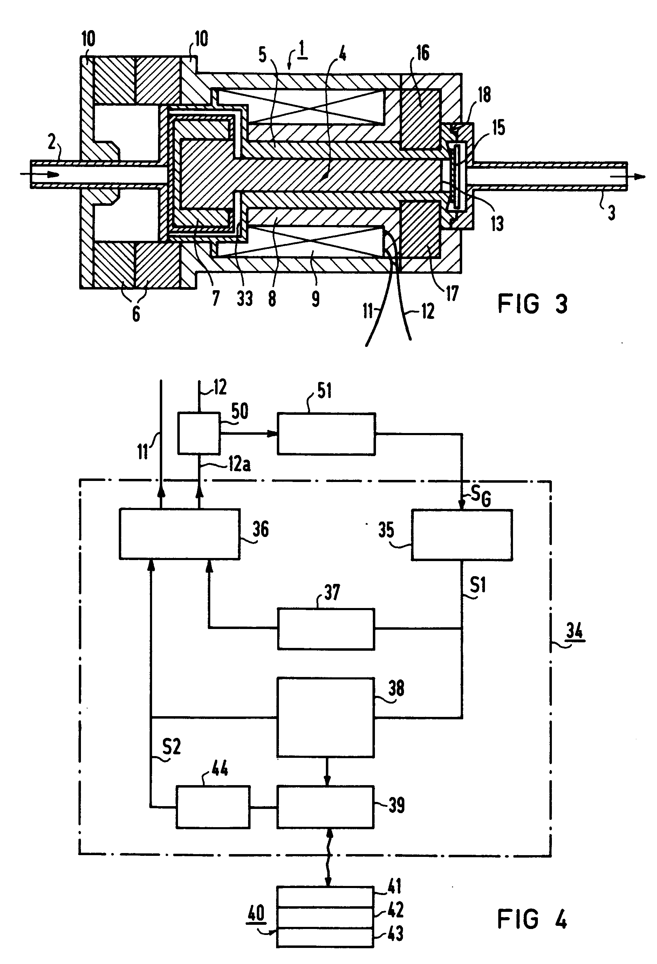

Eine einfachere Art der Funktionsüberwachung einer solchen Pumpe ist in der FIG 3, 4 und 6 dargestellt. Dabei entfällt der Geräuschsensor zu Gunsten einer rein elektrischen Überwachung der Statorwicklung 9. Die Pumpe selbst kann, wie in FIG 3 ersichtlich, dabei kürzer und damit raumsparender ausgeführt werden, was vor allem bei Implantaten eine wesentliche Rolle spielt. Eine solche Anordnung ermöglicht allerdings im Gegensatz zur vorbeschriebenen lediglich eine Überwachung des Anschlagzeitpunktes des Kolbens an der Stirnwand 33 der Zylinderwand 5 sowie die Überwachung der Dauer und der Geschwindigkeit der Kolbenbewegung. Aber auch diese beiden Größen erlauben eine für die meisten Anwendungsfälle ausreichende Überwachung der Pumpenfunktion.

Aus FIG 4 ist zu entnehmen, daß die Steuer- und Überwachungsanordnung mit derjenigen nach FIG 2 identisch ist. Der einzige Unterschied besteht darin, daß das Eingangssignal $S_G$ des Amplituden-Schwellenwertdiskriminators 35 nicht aus einem Geräuschsensor abgeleitet ist, sondern aus dem Stromkreis der Statorwicklung 9. Dazu wird in die Zuleitung 12 des Statorwicklungsstromkreises ein Strommesser 50 eingeschaltet, dessen stromproportionales Ausgangssignal einer Auswerteelektronik 51 zugeleitet wird. In dieser Auswerteelektronik wird das Ausgangssignal des Strommessers 50 differenziert und dieses differenzierte Signal dem Eingang des Amplituden-Schwellenwertdiskriminators 35 zugeführt.

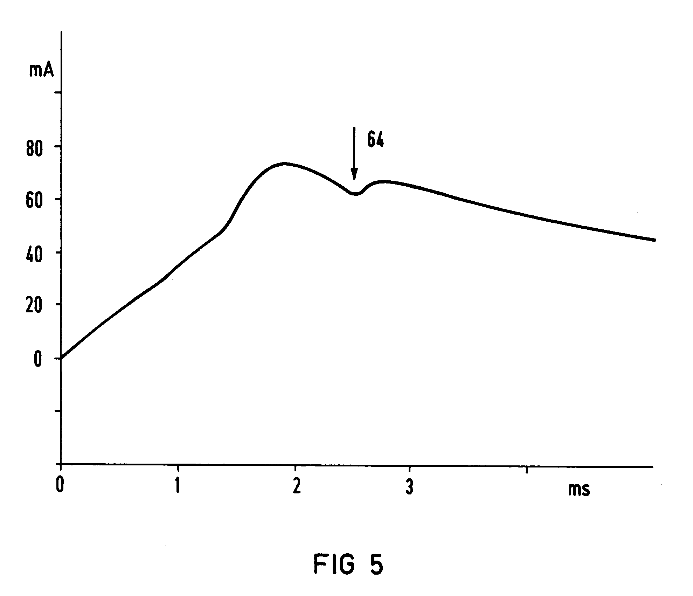

In FIG 5 ist der Stromverlauf durch die Statorwicklung 9 in Abhängigkeit von der Zeit dargestellt, wie er sich bei normaler Funktion der Pumpe ergibt. Dabei markiert der Pfeil 64 den Anschlag des Kolbens. Dies führt - wie ersichtlich - zu einem deutlichen Stromeinbruch, der sich durch Differentiation des Stromsignals leicht erfassen läßt. Dies geschieht in der Auswerteelektronik 51 und führt zu einem Ausgangssignal $S_G$, das sich in der bereits beschriebenen Weise in der Steuer- und Überwachungsanordnung 34 weiter verarbeiten läßt.

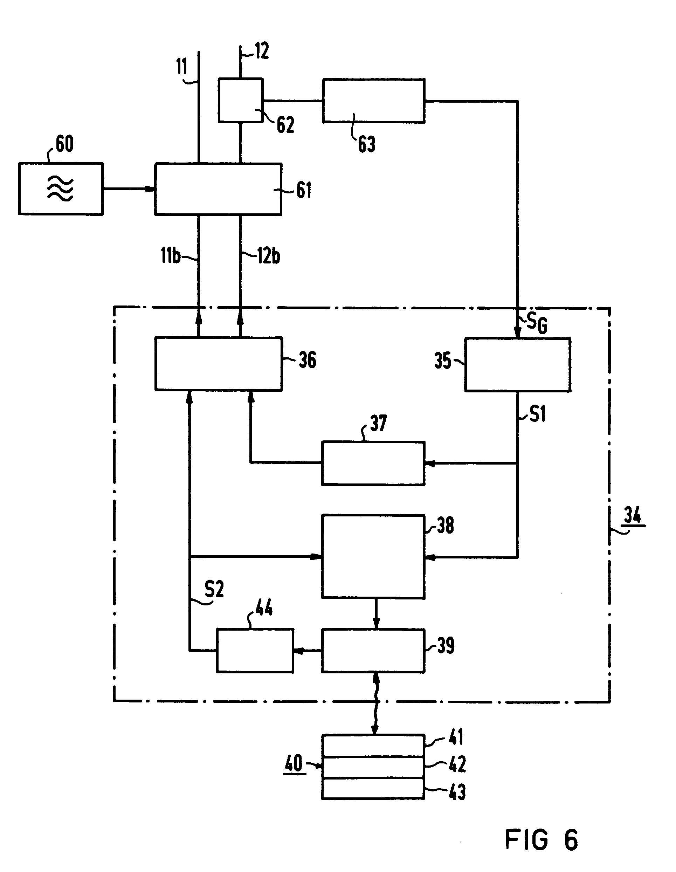

In einer weiteren Ausführungsform nach FIG 6 wird anstelle des Stroms die Induktivität der Statorwicklung 9 gemessen. Dazu wird der Erregergleichspannung eine von einem Generator 60 erzeugte Wechselspannung einer im Stromkreis 11, 12 der Statorwicklung angeordneten Mischstufe 61 zugeführt, die diese Wechselspannung der Erregergleichspannung überlagert. Die Größe des durch die Statorwicklung 9 fließenden Wechselstroms ist von der Induktivität der Statorwicklung abhängig. Dieser Wechselstrom wird in einem Strommesser 62 gemessen. Durch die Bewegung des Kolbens 4 ändert sich die Induktivität der Statorwicklung 9 durch Änderung des magnetischen Flusses in deren magnetischem Kreis. Nach dem Anschlag bleibt die Induktivität der Wicklung hingegen konstant. Der Zeitpunkt des Konstantwerdens läßt sich wiederum durch Differentiation des gleichgerichteten Wechselstromsignals erfassen. Dies geschieht in der Auswerteschaltung 63, deren Ausgangssignal $S_G$ wird wiederum dem Eingang des Amplituden-Schwellenwertdiskriminators 35 zugeführt und in der Steuer- und Überwachungsanordnung 34 wie beschrieben verarbeitet.

**Patentansprüche**

1. Dosiergerät zum gesteuerten Injizieren von Flüssigkeiten aus einem Vorratsbehälter in einen Organismus mittels einer elektrisch steuerbaren Kolbenpumpe (1) mit einem ruhenden Teil (Zylinder 5) und einem gegenüber diesem elektromagnetisch angetriebenen beweglichen Teil (Kolben 4) sowie mit Mitteln für die Funktionssteuerung (40) und Funktionsüberwachung (34) des Dosiergerätes, **dadurch gekennzeichnet,** daß als Mittel für die Funktionsüberwachung eine elektronische Meßanordnung (22, 23, 24, 50, 51, 60, 61, 62, 63) für die indirekte Erfassung der Bewegung des Kolbens (4) der Kolbenpumpe durch Messung des zeitlichen Verlaufs der den Kolben (4) antreibenden elektrischen Eingangsenergie oder der von diesem bewirkten Flüssigkeitsströmung vorhanden ist, deren elektrische Ausgangssignale ($S_G$) einer Steuer- und Überwachungsanordnung (34) zugeführt sind, bestehend aus einem elektronischen Schwellenwertdiskriminator (35) für die Analyse des zeitabhängigen Verlaufs der vom Mittel für die Funktionsüberwachung gelieferten, dem Bewegungsverlauf und/oder der Bewegungsdauer des Kolbens entsprechenden elektrischen Ausgangssignale ($S_G$) sowie Schaltungsmittel (40) für die Erzeugung von Steuer- und/oder Alarmsignalen in Abhängigkeit von vorgebbaren Signalformen der Ausgangssignale ($S_G$) oder bei Abweichung des Signalverlaufs oder der Signaldauer der Ausgangssignale ($S_G$) von vorgebbaren Soll-Werten.

2. Dosiergerät nach Anspruch 1, **dadurch gekennzeichnet,** daß als Schaltungsmittel für die Erzeugung von Steuer- und/oder Alarmsignalen eine Abschalteinrichtung (37) benutzt ist, die auf den beim Anschlagen des Kolbens (4) an der Zylinderwand (33) am Ausgang des Schwellenwertdiskriminators (35) entstehenden Signalverlauf anspricht und den elektrischen Stromkreis (11, 12) für das Antriebsmittel (9) des Kolbens (4) bis zu dem nächstfolgenden Einschaltkommando unterbricht.

3. Dosiergerät nach Anspruch 2, **dadurch gekennzeichnet,** daß zusätzlich zu der Abschalteinrichtung (37) eine Fehlererfassungseinrichtung (38) vorhanden ist, der das Ausgangssignal ($S_1$) des Schwellenwertdiskriminators (35) zugeführt ist, deren Signalausgang mit einer Telekommunikationselektronik (39) verbunden ist, welche über Telekommunikationsmittel mit einem extrakorporalen Programmiergerät (40) in Verbindung steht, das aus einer Telekommunikationselektronik (41), einem Bedienteil (42) und einer Anzeige (43) für von der Fehlererfassungseinrichtung(38) ermittelte Funktionszustände ausgestattet ist, insbesondere mit einer akustischen, optischen und/oder sensorischen Alarmanzeige für fehlerhafte Funktionszustände und mit deren Bedienteil telemetrisch eine Pumpratensteuereinrichtung (44) programmierbar ist, deren ausgangsseitige Signale ($S_2$) der Fehlererfassungseinrichtung (38) und der Pumpenansteuerelektronik (36) zugeführt sind.

4. Dosiergerät nach Anspruch 1, 2 oder 3, **dadurch gekennzeichnet,** daß im Stromkreis (11, 12) der Statorwicklung (9) für den Antrieb des Kolbens (4) der Kolbenpumpe (1) ein Strommesser (50) eingeschaltet ist, der über eine Auswertelektronik (51) mit dem Eingang des Schwellenwertdiskriminators (35) verbunden ist.

5. Dosiergerät nach Anspruch 1, 2 oder 3, **dadurch gekennzeichnet,** daß der Gleichstrom des Erregerstromkreises (11, 12) der Statorwicklung (9) für den Antrieb des Kolbens (4) durch einen mit Hilfe eines Generators (60) erzeugten Wechselstrom überlagert ist und dessen Größe von einem im Erregerstromkreis (11, 12) der Statorwicklung (9) angeordneten Strommesser (62) gemessen und über eine Auswerteschaltung (63) dem Schwellenwertdiskriminator zugeführt ist.

6. Dosiergerät nach Anspruch 1, 2 oder 3, **dadurch gekennzeichnet,** daß der Pumpe ein (1) Geräuschsensor (20) zum Erfassen der Pumpgeräusche zugeordnet ist, der die Pumpgeräusche in elektrische Ausgangssignale ($S_G$) umformt und diese dem Schwellenwertdiskriminator (35) zuführt.

7. Dosiergerät nach Anspruch 6, **dadurch gekennzeichnet,** daß der Geräuschsensor (20) konzentrisch zum Medikamenten-Auslaßkanal (3) angeordnet ist.

8. Dosiergerät nach Anspruch 6 oder 7, **dadurch gekennzeichnet,** daß als Geräuschsensor (20) ein piezokeramischer Körper (22) mit Elektroden (23, 24) sowie eine damit verbundene Koppelmasse (21) als Bezugsmasse umfaßt und an den Elektroden (23, 24) die elektrischen Ausgangssignalleitungen (27, 28) angeschlossen sind.

9. Dosiergerät nach Anspruch 8, **dadurch gekennzeichnet,** daß der piezokeramische Körper (22) als Ringscheibe ausgebildet ist, die so mittels eines Flansches (19) an der Stirnfläche (18) eines das bewegte Pumpteil beinhaltenden Gehäuses (5) angeordnet ist, das die Normale der Ringscheibe in Pumplängsrichtung zeigt.

10. Dosiergerät nach einem der Ansprüche 6 bis 9, **dadurch gekennzeichnet,** daß der Geräuschsensor (20) in einem Frequenzbereich arbeitet, der denjenigen Pumpgeräuschen entspricht, die anzeigen, wenn ein Ansaug- und/oder Ausstoßvorgang während eines Pumpzyklus beendet wurde.

**Claims**

1. Dosing device for the controlled injection of fluids from a reservoir into an organism by means of an electrically controllable piston pump (1) having a resting part (cylinder 5) and a moving part (piston 4) which is electromagnetically driven with respect to the former, and having means for controlling (40) and monitoring (34) the function of the dosing device, characterised in that an electronic measuring arrangement (22, 23, 24, 50, 51, 60, 61, 62, 63) for indirectly sensing the movement of the piston (4) of the piston pump by measuring the variation with time of the electrical input energy driving the piston (4) or of the fluid flow caused by the latter is present as means for monitoring the function, the electrical output signals ($S_G$) of which are supplied to a control and monitoring arrangement (34) consisting of an electronic threshold value discriminator (35) for analysing the time-dependent variation of the electrical output signals ($S_G$) supplied by the means for monitoring the function and corresponding to the variation of the movement and/or the duration of the movement of the piston and circuit means (40) for generating control and/or alarm signals as a function of predeterminable signal shapes of the output signals ($S_G$) or when the signal variation or the signal duration of the output signals ($S_G$) deviates from predeterminable nominal values.

2. Dosing device according to Claim 1, characterised in that a disconnecting device (37), which responds to the signal variation produced at the output of the threshold value discriminator (35) when the piston (4) impinges against the cylinder wall (33) and interrupts the electrical circuit (11, 12) for the drive means (9) of the piston (4) until the next switch-on command follows, is used as circuit means for generating control and/or alarm signals.

3. Dosing device according to Claim 2, characterised in that, in addition to the disconnecting device (37), there is an error detection device (38) to which the output signal ($S_1$) of the threshold value discriminator (35) is supplied and a signal output of which is connected to telecommunication electronics (39) which are connected via telecommunication means with an extracorporeal programming device (40) which is equipped with telecommunication electronics (41), an operating section (42) and a display (43) for functional states determined by the error detection device (38), preferably with an acoustical, optical and/or sensing alarm indicator for faulty functional states and with the operating section of which a pump rate control device (44) can be telemetrically programmed, the output signals ($S_2$) of which are supplied to the error detection device (38) and the pump drive electronics (36).

4. Dosing device according to Claim 1, 2 or 3, characterised in that a current meter (50), which is connected via evaluating electronics (51) to the input of the threshold value discriminator (35), is inserted into the circuit (11, 12) of the stator winding (9) for driving the piston (4) of the piston pump (1).

5. Dosing device according to Claim 1, 2 or 3, characterised in that on the direct current of the exciter circuit (11, 12) of the stator winding (9) for driving the piston (4) an alternating current is superimposed which is generated with the aid of a generator (60), the magnitude of which is measured by a current meter (62) arranged in the exciter circuit (11, 12) of the stator winding (9) and is supplied via an evaluating circuit (63) to the threshold value discriminator.

6. Dosing device according to Claim 1, 2 or 3, characterised in that the pump (1) is associated with a noise sensor (20) for detecting the pump noises which converts the pump noises into electrical output signals ($S_G$) and supplies these to the threshold value discriminator (35).

7. Dosing device according to Claim 6, characterised in that the noise sensor (20) is arranged concentrically with respect to the medicament outlet channel (3).

8. Dosing device according to Claim 6 or 7, characterised in that the noise sensor (20) comprises a piezoceramic body (22) with electrodes (23, 24) and a coupling earth (21) connected thereto as reference earth and the electrodes (23, 24) are connected to the electrical output signal lines (27, 28).

9. Dosing device according to Claim 8, characterised in that the piezoceramic body (22) is constructed as an annular disc which is arranged by means of a flange (19) at the front face (18) of a case (5) containing the moving pump part, in such a manner that the normal of the annular disc points in the longitudinal direction of the pump.

10. Dosing device according to one of Claims 6 to 9, characterised in that the noise sensor (20) operates within a frequency range which corresponds to the pump noises which indicate when an intake and/or ejection process has been ended during a pump cycle.

**Revendications**

1. Dispositif de dosage pour l'injection commandée de liquides dans un organisme à partir d'un réservoir, à l'aide d'une pompe à piston (1) commandable électriquement, comportant une partie fixe (cylindre 5) et une partie mobile (piston 4) entraînée par voie électromagnétique par rapport à la partie fixe, ainsi que des moyens pour la commande du fonctionnement (40) et le contrôle du fonctionnement (34) du dispositif de dosage, caractérisé par le fait qu'il est prévu, comme moyens pour contrôler le fonctionnement, un dispositif de mesure électronique (22,23,24,50,51,60,61,62,62) pour la détermination indirecte du déplacement du piston (4) de la pompe à piston, par mesure de la variation dans le temps de l'énergie électrique d'entrée entraînant le piston (4) ou de l'écoulement de liquide provoqué par ce piston, et dont les signaux électriques de sortie ($S_G$) sont envoyés à un dispositif de commande et de contrôle (34), constitué par un discriminateur électronique à valeur de seuil (35) pour l'analyse de la variation, en fonction du temps, des signaux électriques de sortie ($S_G$) délivrés par les moyens de contrôle du fonctionnement et correspondant à l'allure et/ou la durée de déplacement du piston ainsi que des moyens 40) formant circuit pour la production de signaux de commande et/ou d'alarme en fonction de formes pouvant être prédéterminées des signaux de sortie ($S_G$) ou, dans le cas d'un écart de l'allure ou de la durée des signaux de sortie ($S_G$), en fonction de valeurs de consigne pouvant être prédéterminées.

2. Dispositif de dosage suivant la revendication 2, caractérisé par le fait qu'on utilise comme moyens formant circuit pour la production de signaux de commande et/ou d'alarme, un dispositif de débranchement (37), qui répond à l'allure du signal qui apparaît à la sortie du discriminateur à valeur de seuil (35), lorsque le piston (4) vient s'appliquer contre la paroi (33) du cylindre, et interrompt le circuit électrique (11,12) prévu pour les moyens d'entraînement (9) du piston (4), jusqu'à l'ordre de branchement immédiatement suivant.

3. Dispositif de dosage suivant la revendication 2, caractérisé par le fait qu'en plus du dispositif de débranchement (37), il est prévu un dispositif de détection d'erreurs (38), auquel est envoyé le signal de sortie ($S_1$) du discriminateur à valeur de seuil (35), dont la sortie du signal est raccordée à un système électronique de télécommunication (39), qui est relié, par l'intermédiaire de moyens de télécommunication, à un dispositif extracorporel de programmation (40), qui est constitué par un système électronique de télécommunication (41), une partie de commande (42) et un dispositif d'affichage (43) pour l'affichage d'états de fonctionnement déterminés par le dispositif de détection d'erreurs (38), notamment du dispositif acoustique, optique et/ou sensoriel d'affichage d'alarme pour des états de fonctionnement erronés et à l'aide de la partie de commande duquel peut être programmé, de façon télémétrique, un dispositif (44) de commande du débit de la pompe, dont les signaux ($S_2$) présents côté sortie sont envoyés au dispositif de détection d'erreurs (38) dans le système électronique (36) de commande de la pompe.

4. Dispositif de dosage suivant la revendication 1, 2 ou 3, caractérisé par le fait que dans le circuit (11,12) de l'enroulement statorique (9) prévu pour le dispositif d'entraînement du piston (4) de la pompe à piston (1) est branché un ampèremètre (50) qui est raccordé, par l'intermédiaire d'un circuit électronique d'évaluation (51), à l'entrée du discriminateur à valeur de seuil (35).

5. Dispositif de dosage suivant la revendication 1, 2 ou 3, caractérisé par le fait qu'au courant continu du circuit d'excitation (11, 12) de l'enroulement statorique (9) prévu pour l'entraînement du piston (4) est superposé un courant alternatif produit par un générateur (60) et dont l'intensité est mesurée par un ampèremètre (62) disposé dans le circuit d'excitation (11,12) de l'enroulement statorique (9) et est envoyée, par l'intermédiaire d'un circuit d'évaluation (63), au discriminateur à valeur de seuil.

6. Dispositif de dosage suivant la revendication 1, 2 ou 3, caractérisé par le fait qu'à la pompe (1) est associé un détecteur de bruits (20) servant à détecter les bruits de la pompe et qui convertit les bruits de la pompe en des signaux électriques de sortie ($S_G$) et envoie ces signaux au discriminateur à valeur de seuil (35).

7. Dispositif de dosage suivant la revendication 6, caractérisé par le fait que le détecteur de bruits (20) est disposé concentriquement autour du canal (3) de sortie du médicament.

8. Dispositif de dosage suivant la revendication 6 ou 7, caractérisé par le fait qu'il est prévu comme capteur de bruits (20) un corps piézocéramique (22) comportant des électrodes (22,23) ainsi qu'une masse de couplage (21) formant masse de référence et raccordée à ce corps et que les conducteurs électriques (27,28) de transmission des signaux de sortie sont raccordés aux électrodes (23,24).

9. Dispositif de dosage suivant la revendication 8, caractérisé par le fait que le corps piézocéramique (22) est réalisé sous la forme d'un disque annulaire, qui est appliqué, au moyen d'une bride (19), contre la face frontale (18) d'un carter (5) contenant la partie mobile de la pompe et qui oriente la normale du disque annulaire dans la direction longitudinale de la pompe.

10. Dispositif de dosage suivant l'une des revendications 6 à 9, caractérisé par le fait que le détecteur de bruits (20) travaille dans une gamme de fréquences, qui correspond aux bruits de la pompe, qui indiquent le moment où un processus d'aspiration et/ou un processus de refoulement est terminé pendant un cycle de la pompe.

FIG 1

FIG 2

FIG 3

FIG 4

FIG 5

FIG 6